# DEMANDE DE BREVET EUROPEEN

(11) **EP 0 850 640 A1**
(43) Date de publication de la demande: **01.07.1998**
(21) Numéro de dépôt: 97402923.3
(22) Date de dépôt: 03.12.1997
(51) Int. Cl.: A61K 7/48

(54) **Composition de soin contenant un gel d'organopolysiloxane solide élastomère**

(30) Priorité: 24.12.1996 FR 9615986
(71) Demandeur: L'OREAL, 75008 Paris (FR)
(72) Inventeur: Bara, Isabelle, 75013 Paris (FR)
(74) Mandataire: Lhoste, Catherine

(57) **Abrégé**

L'invention se rapporte à une composition huileuse de soin contenant comme gélifiant au moins un organopolysiloxane solide élastomérique partiellement réticulé et une phase grasse associée à ce gélifiant, renfermant au moins une huile polaire et au moins un co-solvant à structure siliconée comportant une chaîne pendante et/ou en bout de structure siliconée, cette chaîne étant linéaire ou ramifiée et comportant de 3 à 12 atomes de carbone. Ce co-solvant est en particulier volatile à température ambiante, conférant de la fraîcheur à l'application.

## Description

La présente invention se rapporte à une composition de soin et/ou de traitement de la peau, y compris du cuir chevelu, et/ou des lèvres des êtres humains, contenant un gel d'organopolysiloxane comme gélifiant. Cette composition est, utilisable dans le domaine cosmétique ou dermatologique. Plus spécialement cette composition se présente sous forme d'une gel anhydre homogène renfermant une ou plusieurs huiles traitantes. En particulier, cette composition permet l'hydratation et la nutrition de la peau. Elle lui assure de la souplesse et de la douceur.

Les gels anhydres à base d'organosiloxane élastomérique réticulé, comme décrits dans le document US -A 4 987 169, sont connus pour être des produits destinés à être appliqués sur la peau, présentant de bonnes qualités cosmétiques comme la douceur, la matité et un toucher non gras.

Les gels les plus homogènes sont obtenus en faisant gonfler des résines avec des huiles de nature siliconées, comme les polydiméthylsiloxanes (PDMS) linéaires et les cyclométhicones, ou des huiles apolaires comme les dérivés de paraffines issues du pétrole, volatiles ou non, ou de synthèse tel que le perhydrosqualane.

Ces huiles, bien que conférant certaines propriétés à la peau (propriété anti-déshydratante par effet protecteur ou en s'opposant à la perte insensible en eau - PIE-), ne sont en aucun cas « traitantes» pour la peau. En effet les huiles de soin sont plutôt d'origine végétale et de nature polaire, telles que les triglycérides.

Malheureusement ces huiles polaires sont très difficilement gélifiables par les silicones élastomériques ; le mélange est soit infaisable, ou s'il se fait, est instable au cours du temps, d'aspect granuleux et opaque. En outre, ces huiles, employées telles quelles, sont trop fluides pour être appliquées sur la peau de façon aisée. Par ailleurs, elles apportent un toucher gras peu confortable, ainsi qu'un effet brillant à la peau.

L'invention a justement pour objet une composition de soin et/ou de traitement de la peau et/ou des lèvres permettant de remédier à ces inconvénients. De façon surprenante, le demandeur a trouvé que l'utilisation d'alkyl diméthicone fluide, appelé aussi huile de silicone à chaîne alkyle, permettait de rendre compatible les huiles traitantes polaires avec les résines organopolysiloxanes et de réaliser des gels stables, translucides, voire transparents, permettant de véhiculer sans contrainte et de manière agréable, des huiles traitantes.

L'invention s'applique non seulement aux produits de soin et/ou de traitement de la peau, y compris du cuir chevelu, des muqueuses comme les lèvres et l'intérieur des paupières, mais aussi aux produits de maquillage des lèvres ayant des propriétés de soin et/ou de traitement, ainsi qu'aux produits de maquillage de la peau, aussi bien du visage que du corps, ayant une valence soin et/ou traitante.

De façon plus précise, l'invention a pour objet une composition contenant au moins un organopolysiloxane solide élastomérique partiellement réticulé, comme gélifiant, associé à une phase grasse renfermant au moins une huile polaire et au moins un co-solvant à structure siliconée comportant au moins une chaîne alkyle pendante et/ou en bout de structure siliconée, cette chaîne étant linéaire ou ramifiée et comportant de 3 à 12 atomes de carbone.

Cette composition peut être utilisée telle quelle ou bien être incorporée dans une composition plus complexe.

Par « élastomérique » on entend un matériau souple, déformable ayant des propriétés viscoélastiques et présentant notamment la consistance d'une éponge ou d'une sphère souple.

Les organopolysiloxanes élastomériques de la composition selon l'invention présentent un remarquable pouvoir gélifiant d'huile. Ils ne sont pas desséchants pour la peau et apportent de bonnes propriétés cosmétiques. Ces nouveaux élastomères conduisent à des compositions confortables à l'application, douces et non collantes au toucher. Cette douceur est due notamment à la texture des organopolysiloxanes.

De plus, ces compositions, grâce à la présence d'huile polaire, présentent des propriétés traitantes et/ou de soin.

La composition de l'invention peut se présenter sous forme de pâte, de solide, de crème. Elle peut être une émulsion huile-dans-eau ou eau-dans-huile, un gel anhydre, solide ou souple. De préférence, elle se présente sous forme de gel anhydre translucide ou transparent.

De façon avantageuse, le ou les co-solvants sont des huiles volatiles, c'est-à-dire des huiles susceptibles de s'évaporer, à température ambiante notamment de la peau et/ou des lèvres. Dans ce cas, les compositions de l'invention, après évaporation de l'huile ou des huiles volatiles, conduisent à un film homogène et uniforme, ayant une texture légère et conférant de la fraîcheur, et ce malgré la présence de deux matériaux au départ incompatibles.

Les organopolysiloxanes élastomériques de la compsosition selon l'invention sont en général partiellement ou totalement réticulés et de structure tridimensionnelle. Inclus dans une phase grasse, ils se transforment, selon le taux de phase grasse utilisé, d'un produit d'aspect spongieux lorsqu'ils sont utilisés en présence de faibles teneurs en phase grasse en un gel plus ou moins homogène, en présence de quantités de phase grasse plus élevées. La gélification de la phase grasse par ces élastomères peut être totale ou partielle.

Les élastomères de la composition de l'invention sont véhiculés généralement sous forme de gel constitué d'un organopolysiloxane élastomérique de structure tridimensionnelle, inclus dans au moins une huile hydrocarbonée et/ou une huile siliconée.

Les organopolysiloxanes élastomériques de la composition selon l'invention peuvent être choisis parmi les polymères réticulés décrits dans la demande EP-A-0295886. Selon cette demande, ils sont obtenus par réaction d'addition et de réticulation, en présence d'un catalyseur du type platine, d'au moins :
- (a) un organopolysiloxane ayant au moins deux groupes alcényle inférieurs en C₂-C₆ par molécule ; et
- (b) un organopolysiloxane ayant au moins deux atomes d'hydrogène liés à un atome de silicium par molécule.

Les organopolysiloxanes élastomériques de la composition selon l'invention peuvent aussi être choisis parmi ceux décrits dans le brevet US 5 266 321. Selon ce brevet, ils sont choisis notamment parmi :
- i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle tel que méthyle, éthyle ou propyle, un aryle tel que phényle ou tolyle, un groupe aliphatique insaturé tel que vinyle, le rapport en poids des motifs R₂SiO sur les motifs RSiO_{1,5} allant de 1/1 à 30/1 ;
- ii) les organopolysiloxanes insolubles et gonflables dans une huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

Les organopolysiloxanes objet de l'invention sont par exemple ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels déjà constitués (KSG15, KSG17, KSG16, KSG18 de Shin-Etsu, Gransil SR 5CYC gel, Gransil SR DMF 10 gel, Gransil SR DC 556 gel, SF 1204 et JK 113 de General Electric. On peut aussi utiliser un mélange de ces produits commerciaux.

De façon préférentielle, le ou les organopolysiloxanes sont présents, en matière active, à une concentration allant de 0,1 à 80 % du poids total de la composition et de préférence de 2 à 60 %.

Les silicones volatiles à chaîne alkyle répondent notamment à la formule (1) suivante :

R¹(CH₃)₂Si-O-{-Si-(CH₃)R²-O-}ₙ-{-Si-(CH₃)₂-O-}ₘ-Si(CH₃)₂R¹

où R¹ et R² sont indépendamment H, méthyle ou une chaîne alkyle linéaire ou ramifiée ayant de 3 à 10 atomes de carbone, n et m étant des entiers allant de 0 à 10, à condition que si R¹ vaut H ou méthyle, n soit différent de 0 et R² représente une chaîne alkyle de 3 à 10 atomes.

Avantageusement les silicones volatiles de formule (1) ont une masse moléculaire allant de 290 à 3 000 et une volatilité ou taux d'évaporation Nv généralement compris entre 150 et 500 secondes (temps correspondant à l'évaporation de 0,2 ml de silicone volatile à 23°C dans une atmosphère stable à 50 % d'humidité relative).

Comme silicones volatiles alkyle utilisables dans l'invention ; on peut citer les alkyl heptaméthyltrisiloxanes avec un groupe alkyle en C₄, C₅, C₆, C₇ et C₈ comme par exemple l'hexyl heptaméthyltrisiloxane de formule : (CH₃)₃-Si-O-Si(CH₃)(C₆H₁₃)-O-Si(CH₃)₃ ; l'octyl heptaméthyltrisiloxane de formule : (CH₃)₃-Si-O-Si(CH₃)(C₈H₁₅)-O-Si(CH₃)₃ ; et leurs mélanges.

De préférence, la ou les huiles de silicone volatiles alkylées représentent de 10 à 99 % en poids, par rapport aux huiles polaires et mieux de 20 % à 80 %.

L'emploi d'un co-solvant selon l'invention permet de rendre compatible l'organo-polysiloxane élastomérique avec les huiles polaires. De plus, l'emploi d'un co-solvant volatil présente l'avantage d'une élimination rapide, laissant subsister sur la zone d'application de la composition uniquement l'organopolysiloxane et l'huile polaire.

Comme huiles polaires utilisables dans l'invention, on peut citer notamment :
- les huiles hydrocarbonées végétales telles que les triglycérides liquides d'acides gras, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R₉COOR₁₀ dans laquelle R₉ représente le reste d'un acide gras supérieur comportant de 7 à 19 atomes de carbone et R₁₀ représente une chaîne hydrocarbonée ramifiée contenant de 3 à 20 atomes de carbone comme par exemple l'huile de Purcellin (octanoate de cétostéaryle) ;
- les esters et les éthers de synthèse comme le myristate d'isopropyle, des octanoates, décanoates ou ricinoléates d'alcools ou de polyalcools ;
- des alcools gras comme l'actyl dodécanol ou l'alcool oléique ;
- leurs mélanges.

De préférence les huiles polaires de l'invention sont des huiles végétales hydrocarbonées à forte teneur en triglycérides constitués d'esters d'acides gras et glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat. Ces huiles végétales ont la particularité d'être liquides à température égale ou inférieure à 25°C.

Il est éventuellement possible d'ajouter aux huiles polaires des huiles non polaires ou apolaires comme les huiles siliconées telles que les polyméthylsiloxanes volatiles ou non linéaires ou cycliques, liquides ou pâteux à température ambiante ; les hydrocarbures linéaires ou ramifiés d'origine synthétique ou minérale comme les huiles de paraffine volatiles ou non et leurs dérivés, la vaseline, les polydécènes, le polyisobutène hydrogéné tel que le parléam.

Les huiles polaires représentent de 0,2 à 80 % du poids total de la composition, de préférence de 1 à 50 %. Les huiles non polaires ou apolaires représentent de 0 à 80 % du poids de la composition et mieux de 0 à 30 %.

La composition de l'invention peut comprendre, en outre, tout additif usuellement utilisé dans le domaine concerné, tel que l'eau, des colorants hydrosolubles ou liposolubles, des antioxydants, des huiles essentielles, des conservateurs, des neutralisants, des polymères liposolubles notamment hydrocarbonés tels que les polyalkylènes, des actifs cosmétiques ou dermatologiques comme par exemple des émollients, des hydratants, des vitamines, des actifs antirides, des acides gras essentiels, des filtres solaires lipophiles, des gélifiants de phase aqueuse. Ces additifs peuvent être présents dans la composition à raison de 0 à 20% du poids total de la composition et mieux de 0 à 10%.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

La composition selon l'invention se présente avantageusement sous forme d'un gel anhydre qui peut être translucide, voire transparent.

La composition selon l'invention peut se présenter sous la forme d'une composition dermatologique ou de soin de la peau, ou sous forme d'une composition de protection solaire ou de démaquillage. Elle se présente alors sous forme non colorée, contenant éventuellement des actifs cosmétiques ou dermatologiques. Elles peuvent alors être utilisées comme base de soin pour la peau ou les lèvres (baumes à lèvres, protégeant les lèvres du froid et/ou du soleil et/ou du vent).

La composition de l'invention peut également se présenter sous la forme d'un produit coloré de maquillage de la peau, en particulier un fond de teint, un blush, un fard à joues ou à paupières, ou de maquillage des lèvres comme un rouge à lèvres, présentant des propriétés de soin ou de traitement.

Bien entendu la composition de l'invention doit être cosmétiquement ou dermatologiquement acceptable, à savoir non toxique et susceptible d'être appliqué sur la peau ou les muqueuses (lèvres, intérieur des paupières) d'êtres humains.

La composition de l'invention peut comprendre une phase particulaire, généralement présente à raison de 0 à 35 % du poids total de la composition, de préférence de 5 à 25 %, et qui peut comprendre des pigments et/ou des nacres et/ou des charges habituellement utilisés dans les compositions cosmétiques.

La composition selon l'invention peut être fabriquée par les procédés connus, généralement utilisés dans le domaine cosmétique ou dermatologique.

L'invention a encore pour objet un procédé cosmétique de soin ou de traitement de la peau ou des muqueuses des êtres humains, comprenant l'application sur la peau ou les muqueuses de la composition telle que définie ci-dessus.

L'invention est illustrée plus en détail dans les exemples suivants. Les pourcentages sont donnés en poids.

### Exemple 1 : huile de soin du visage

- Huile de tournesol 5,00 %
- Huile de rosier muscat 0,87 %
- Huile de pépins de cassis 0,45 %
- Antioxydant 0,03 %
- N-hexylheptaméthyltrisiloxane vendu par la Société Dow Corning sous la dénomination de DC1731 qsp 100 %
- Mélange de 40 % en poids de polydiméthylsiloxane 6 cst et de 60 % en poids de polydiméthylorganosiloxane partiellement réticulé vendu sous le nom KSG6 par Shin Etsu 50,00 %

*Préparation :* On fait gonfler l'organopolysiloxane dans le mélange d'huiles volatiles et polaires à températures ambiante, en présence de l'antioxydant.

On obtient un gel anhydre translucide et incolore, de texture agréable, qui s'étale bien et de façon homogène.

### Exemple 2 : Dispersion gélifiée de soin de la peau du visage

- Organopolysiloxane élastomérique (KSG6) 10,00 %
- Pemulen TR2 de chez Goodrich (acrylates/C₁₀ -C₃₀ alkyl acrylate crosspolymer - dispersant -) 0,10 %
- Carbopol 980 de chez Goodrich (carboxyvinylique -gélifiant de phase aqueuse -) 0,60 %
- Triéthanolamine (neutralisant) 0,80 %
- Conservateur qs
- N-hexylheptaméthyltrisiloxane vendu par la Société Dow Corning sous la dénomination de DC1731 10,00 %
- Huile d'abricot 10,00 %
- Antioxydant 0,03 %
- Eau qsp 100 %

## Revendications

1. Composition contenant au moins un organopolysiloxane solide élastomérique partiellement réticulé, comme gélifiant, associé à une phase grasse renfermant au moins une huile polaire et au moins un co-solvant à structure siliconée comportant au moins une chaîne alkyle pendante et/ou en bout de structure siliconée, cette chaîne étant linéaire ou ramifiée et comportant de 3 à 12 atomes de carbone.

2. Composition selon la revendication 1, caractérisée en ce que le co-solvant est une huile volatile.

3. Composition selon la revendication 1 ou 2, caractérisée en ce que le co-solvant présente la formule suivante :
R¹(CH₃)₂Si-O-{-Si(CH₃)R²-O-}ₙ{-Si-(CH₃)₂-O-}ₘ-Si(CH₃)₂R¹
où R¹ et R² sont indépendamment H, méthyle ou une chaîne alkyle linéaire ou ramifiée ayant de 3 à 10 atomes de carbone, n et m étant des entiers allant de 0 à 10, à condition que si R¹ vaut H ou méthyle, n soit différent de 0 et R² représente une chaîne alkyle de 3 à 10 atomes.

4. Composition selon l'une quelconque des revendications précédentes, caractérisée en ce que le co-solvant est choisi parmi l'hexyl heptaméthyltrisiloxane, l'octyl heptaméthyltrisiloxane et leurs mélanges.

5. Composition selon l'une quelconque des revendications précédentes, caractérisée en ce que le co-solvant est présent à raison de 10 à 99 % en poids par rapport aux huiles polaires.

6. Composition selon l'une quelconque des revendications précédentes, caractérisée en ce que l'huile polaire est une huile végétale à forte teneur en triglycérides.

7. Composition selon l'une quelconque des revendications précédentes, caractérisée en ce que l'huile polaire est présente à raison de 0,2 à 80 % du poids total de la composition.

8. Composition selon l'une quelconque des revendications précédentes, caractérisée en ce que l'organo-polysiloxane élastomérique est obtenu par réaction d'addition et de réticulation, en présence d'un catalyseur, d'au moins :
(a) un organopolysiloxane ayant au moins deux groupes alcényle inférieurs en C₂-C₆ par molécule ;
(b) un organopolysiloxane ayant au moins deux atomes d'hydrogène liés à un atome de silicium par molécule.

9. Composition selon l'une quelconque des revendications précédentes, caractérisée en ce que l'organopolysiloxane est choisi parmi :
- i) les organopolysiloxanes comprenant des motifs R₂SiO et RSiO_{1,5} et éventuellement des motifs R₃SiO_{0,5} et/ou SiO₂ dans lesquels les radicaux R, indépendamment les uns des autres, représentent un hydrogène, un alkyle, un aryle, un groupe aliphatique insaturé, le rapport en poids des motifs R₂SiO sur les motifs RSiO_{1,5} allant de 1/1 à 30/1 ;
- ii) les organopolysiloxanes insolubles dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol lorsque l'organopolysiloxane est non-cyclique et entre 1 et 50% mol lorsque l'organopolysiloxane est cyclique.

10. Composition selon l'une quelconque des revendications précédentes, caractérisée en ce que l'organopolysiloxane solide élastomérique est présent en matière active en une concentration allant de 0,1 à 80 % du poids total de la composition

11. Composition selon l'une quelconque des revendications précédentes, caractérisée en ce qu'elle contient au moins un actif cosmétique et/ou dermatologique.

12. Composition selon l'une quelconque des revendications précédentes, caractérisée en ce qu'elle se présente sous forme d'une émulsion huile-dans-eau ou eau-dans-huile, d'un gel anhydre solide ou souple.

13. Composition selon l'une quelconque des revendications précédentes, caractérisée en ce qu'elle contient, en outre, au moins une huile non polaire et/ou au moins un additif choisi parmi l'eau, les colorants hydrosolubles ou liposolubles, les antioxydants, les huiles essentielles, les conservateurs, les gélifiants de phase aqueuse, les neutralisants, les polymères liposolubles.

14. Composition selon l'une quelconque des revendications précédentes, caractérisée en ce qu'elle constitue une composition de soin et/ou de traitement de la peau ou des muqueuses, une composition de maquillage de la peau ou des muqueuses avec une valence soin et/ou traitement.

15. Procédé de soin ou de traitement cosmétique de la peau ou des muqueuses des êtres humains, comprenant l'application sur la peau ou les muqueuses de la composition cosmétique selon l'une quelconque des revendications précédentes.
